# EUROPÄISCHE PATENTSCHRIFT

(11) **EP 3 328 390 B1**
(45) Veröffentlichungstag und Bekanntmachung des Hinweises auf die Patenterteilung: **29.09.2021**
(21) Anmeldenummer: 16748057.3
(22) Anmeldetag: 12.07.2016
(51) Int. Cl.: A61K 31/593, A61P 43/00, A61P 39/00, A61K 9/70, A61K 9/00, A61K 9/08, A61K 9/10, A61K 31/198, A61K 47/02, A61K 47/14

(54) **LYSINSALZE VON CHOLECALCIFEROLSULFAT UND DEREN VERWENDUNG ZUR BEHANDLUNG VON VITAMIN D3-MANGEL**
LYSINE SALTS OF CHOLECALCIFEROL SULFATE AND THEIR USE FOR THE TREATMENT OF VITAMIN D3 DEFICIENCY
LYSINE SELS DU SULFATE DE CHOLÉCALCIFÉROL ET LEUR UTILISATION POUR LE TRAITEMENT D'UNE CARENCE EN VITAMINE D3

(30) Priorität: 12.07.2015 DE 102015009022; 15.11.2015 DE 102015014760
(43) Veröffentlichungstag der Anmeldung: 06.06.2018
(73) Patentinhaber: CFSO GmbH, 82538 Geretsried (DE)
(72) Erfinder: SALAMI, Oyewole,Taye, 82515 Wolfratshausen (DE); OBENLAND, Sigrid, 80638 München (DE); CALIEBE, Reinhard, 95182 Döhlau O.T. Tauberlitz (DE)
(74) Vertreter: Obenland, Sigrid
(86) Internationale Anmeldenummer: PCT/EP2016/001201
(87) Internationale Veröffentlichungsnummer: WO 2017/008902

(56) Entgegenhaltungen:
- C. G. PERRINE ET AL: "Adherence to Vitamin D Recommendations Among US Infants", PEDIATRICS, Bd. 125, Nr. 4, 1. April 2010 (2010-04-01) , Seiten 627-632, XP055306727, US ISSN: 0031-4005, DOI: 10.1542/peds.2009-2571
- Sreeramulu Nagubandi ET AL: "Synthesis and Biological Activity of Vitamin D, 3P-Sulfate ROLE OF VITAMIN D.3 SULFATES IN CALCIUM HOMEOSTASIS*", THE JOURNAL OF BlOLOGICAL CHEMISTRY, 10. Juni 1981 (1981-06-10), Seiten 5536-5539, XP055306641, Gefunden im Internet: URL:http://www.jbc.org/content/256/11/5536 .full.pdf#page=1&view=FitH [gefunden am 2016-09-29]
- LEONOR CANCELA ET AL: "Lack of biological activity of vitamin D 3 -3[beta] sulfate during lactation in vitamin D-deficient rats", REPRODUCTION, NUTRITION, DEVELOPMENT, Bd. 27, Nr. 6, 1. Januar 1987 (1987-01-01) , Seiten 979-997, XP055306706, FR ISSN: 0181-1916, DOI: 10.1051/rnd:19870802
- BOULCH N L ET AL: "Cholecalciferol sulfate identification in human milk by HPLC", STEROIDS, ELSEVIER SCIENCE PUBLISHERS, NEW YORK, NY, US, Bd. 39, Nr. 4, 1. April 1982 (1982-04-01), Seiten 391-398, XP023429831, ISSN: 0039-128X, DOI: 10.1016/0039-128X(82)90063-0 [gefunden am 1982-04-01]
- THOMAS A LEBBETTER: "THE CANADIAN MEDICAL ASSOCIATION JOURNAL", THE CANADIAN MEDICAL ASSOCIATION JOURNAL, Bd. 14, Nr. 12, 1. Januar 1924 (1924-01-01), Seiten 1179-1182, XP055306688,

## Beschreibung

Die Erfindung betrifft die Verwendung der pharmazeutisch annehmbaren Cholecalciferolsulfat-Salze L-Lysincholecalciferolsulfat und D,L-Lysincholecalciferolsulfat zur Bekämpfung von Vitamin D₃-Mangel bei Wirbeltieren und insbesondere Menschen durch eine Verabreichungsart, die bewirkt, dass mindestens 5 % der Cholecalciferolsulfat-Salze als solche ohne Metabolisierung vom Verabreichungsort in ein systemisches Flüssigkeitstransportsystem des menschlichen Körpers gelangen, die neuen pharmazeutisch annehmbaren Cholecalciferolsulfat-Salze L-Lysincholecalciferolsulfat und D,L-Lysincholecalciferolsulfat, sowie pharmazeutische Zusammensetzungen für die oben genannten Dosierungsformen Verabreichung, welche diese pharmazeutisch annehmbaren Cholecalciferolsulfat-Salze enthalten.

Die Erfindung wird durch die Ansprüche definiert. Jeglicher Gegenstand, der über den Umfang der Ansprüche hinausgeht, wird lediglich zur Information offenbart.

Es ist bekannt, dass ein großer Teil der Weltbevölkerung an Vitamin-D₃-Mangel leidet.

Auf natürliche Weise wird Vitamin D₃ beim Menschen bekanntlich unter Einwirkung von UV-Licht in der Haut in Form von wasserlöslichem Vitamin D₃-Sulfat (Cholecalciferolsulfat) aus 7-Dehydrocholesterol gebildet. Insbesondere in den Wintermonaten ist es vielen Menschen nicht möglich, sich genügend UV-Licht auszusetzen, damit hinreichende Mengen an Vitamin D3-Sulfat in der Haut gebildet werden können.

Cholecalciferolsulfat ist in Muttermilch und in geringen Mengen auch in anderer Milch enthalten, siehe beispielsweise C. G. PERRINE ET AL: "Adherence to Vitamin D Recommendations Among US Infants", PEDIATRICS, Bd. 125, Nr. 4, 1. April 2010 (2010-04-01), Seiten 627-632. Einige andere Lebensmittel, z.B. Lebertran, enthalten nicht-sulfatiertes fettlösliches Vitamin D₃ (Cholecalciferol). In Nahrungsergänzungsmitteln liegt Vitamin D₃ ebenfalls als fettlösliches Cholecalciferol vor. Diese letztgenannte Form von Vitamin D₃ wird im Körper nicht sulfatiert.

Das Ziel der Erfindung war es dafür zu sorgen, dass dem Körper natürliches Cholecalciferolsulfat auf rasch wirkende oder auch länger anhaltende Art ohne die Notwendigkeit einer Bestrahlung der Haut mit UV-Licht zugeführt wird.

### Zusammenfassung der Erfindung

Demgemäß betrifft die Erfindung die Verwendung der pharmazeutisch annehmbaren Cholecalciferolsulfat-Salze L-Lysincholecalciferolsulfat und D,L-Lysincholecalciferolsulfat, zur Bekämpfung von Vitamin D₃-Mangel bei Wirbeltieren und insbesondere Menschen durch eine Verabreichungsart, die bewirkt, dass mindestens 5 % der Cholecalciferolsulfat-Salze als solche ohne Metabolisierung vom Verabreichungsort in ein systemisches Flüssigkeitstransportsystem des menschlichen Körpers gelangen.

Ferner betrifft die Erfindung die neuen pharmazeutisch annehmbaren Cholecalciferolsulfat-Salze L-Lysincholecalciferolsulfat und D,L-Lysincholecalciferolsulfat, Schließlich betrifft die Erfindung eine pharmazeutische Zusammensetzung, die mindestens eines der pharmazeutisch annehmbaren Cholecalciferolsulfat-Salze L-Lysincholecalciferolsulfat und D,L-Lysincholecalciferolsulfat und ein für die transdermale oder transmukosale Verabreichung oder intradermale, subkutane oder intramuskuläre Injektion geeignetes Vehikel umfasst.

### Kurze Beschreibung der Zeichnung

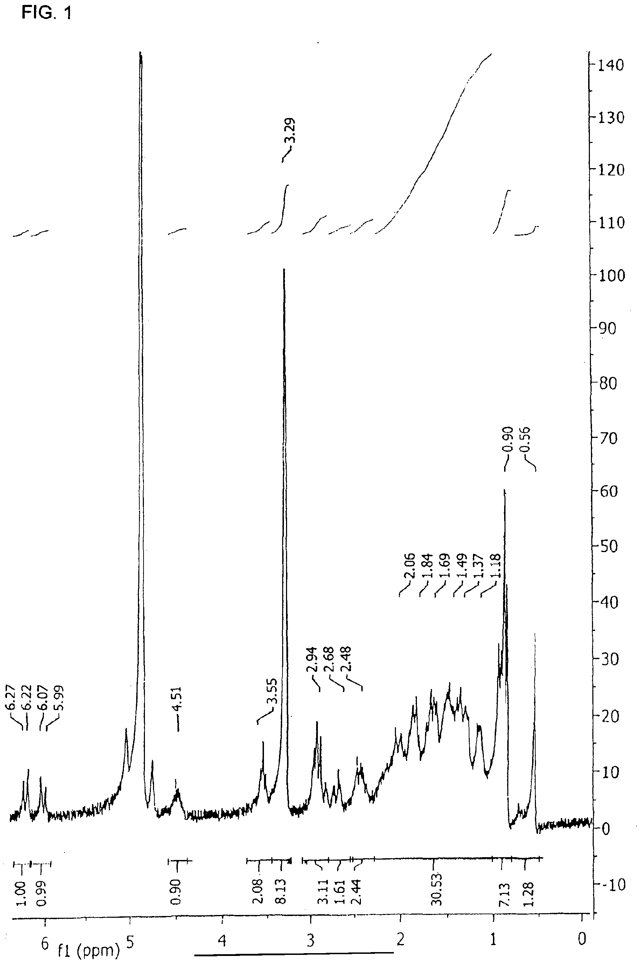

Fig. 1 zeigt das NMR Spektrum (200 MHz; interner Standard: TMS) von L-Lysincholecalciferolsulfat in D₄-Methanol.

### Detaillierte Beschreibung

Es wurde überraschend gefunden, dass Cholecalciferolsulfat-Salze und damit Vitamin D₃ in seiner nativen Form dem Körper auf eine Weise zugeführt werden können, ohne dass sie sofort zum nicht-wasserlöslichen Cholecaliferol oder (25OH)-Cholecalciferol metabolisiert werden. Das Lymphsystem ist in der Lage, die wasserlöslichen Cholecalciferolsulfat-Salze als solche im Körper zu verteilen. Die wasserlöslichen Cholecalciferolsulfat-Salze sind natürlich auch im Blut löslich, können aber dort unter Umständen einem schnellen, z.B. enzymatischen Abbau unterliegen.

Bekämpfung von Vitamin -D₃-Mangel bedeutet hierin Vorbeugung gegen und Behandlung von Vitamin -D₃-Mangel.

Pharmazeutisch annehmbare Cholecalciferolsulfat-Salze bedeutet hierin, dass das Kation dieser Salze auf keinerlei Weise für den menschlichen Körper toxisch ist. Pharmazeutisch annehmbare Kationen sind z.B. Na, Mg, Ca, Zn, Ammonium und protonierte Aminosäuren, wie protoniertes Lysin. Erfindungsgemäß sind jedoch nur die Cholecalciferolsulfat-Salze L-Lysincholecalciferolsulfat und D,L-Lysincholecalciferolsulfat.

Cholecalciferolsulfat-Salze sind in der Regel in Wasser oder in wässrig-alkoholischer Mischung löslich. Beispiele für geeignete pharmazeutisch annehmbare Cholecalciferolsulfat-Salze mit anorganischem Kation sind Cholecalciferolsulfat-Natrium, Cholecalciferolsulfat-Magnesium, Cholecalciferolsulfat-Calcium und Ammoniumcholecalciferolsulfat. Beispiele für geeignete pharmazeutisch annehmbare Cholecalciferolsulfat-Salze mit organischem Kation sind Trimethylammoniumcholecalciferolsulfat und L-Lysincholecalciferolsulfat.

Die erfindungsgemäße Verabreichungsart wird so gewählt, dass sie bewirkt, dass mindestens 5 % des verabreichten Cholecalciferolsulfat-Salzes als solches ohne Metabolisierung vom Verabreichungsort in ein systemisches Flüssigkeitstransportsystem des menschlichen Körpers gelangt. Bevorzugter gelangen, je nach Verabreichungsart, mindestens 10%, 15%, 20%, 25%, 30%, 35%, 40%, 45% oder 50% des verabreichten Cholecalciferolsulfat-Salzes als solches ohne Metabolisierung vom Verabreichungsort in ein systemisches Flüssigkeitstransportsystem des menschlichen Körpers.

Bevorzugt wird das verabreichte Cholecalciferolsulfat-Salz in zumindest einem systemischen Flüssigkeitstransportsystem ausgewählt aus Blut und Lymphe über eine Zeitspanne von mindestens 5 min, mehr bevorzugt mindestens 10, 15, 20 oder sogar 30 min zu weniger als 70%, bevorzugt weniger als 60%, z.B. 50%, 40%, 30%, 20% oder sogar zu 10% oder weniger chemisch verändert (metabolisiert).

Systemische Flüssigkeitstransportsysteme des menschlichen Körpers sind z.B. Blut und bevorzugt Lymphe.

Bevorzugte Verabreichungsarten für die pharmazeutisch annehmbaren Cholecalciferolsulfat-Salze sind die transdermale und transmukosale Verabreichung sowie die intradermale, subkutane und intramuskuläre Injektion, wobei Depot-Injektionen mit verlängerter Wirkstofffreigabe eingeschlossen sind. Perorale Verabreichung (d.h. Aufnahme in den Magen z.B. durch Schlucken), intravenöse und intraarterielle Injektion werden ausdrücklich als Verabreichungswege ausgeschlossen.

Die molare Menge der verabreichten Cholecalciferolsulfat-Salze entspricht im Allgemeinen der molaren Menge, die für Cholecaliferol empfohlen wird.

Die Herstellung der Cholecalciferolsulfat-Salze kann durch Umsetzung von käuflichem Cholecalciferol mit Pyridin-Schwefeltrioxid-Komplex in Pyridin und anschließende Umsetzung mit Triethylamin zum Triethylammoniumcholecalciferolsulfat erfolgen, welches dann in wässriger Lösung durch Zugabe gesättigter Lösungen geeigneter Kationen zur Herstellung von Salzen mit diesen Kationen umgefällt werden kann.

Alternativ kann Cholecaliferol mit Pyridin-Schwefeltrioxid-Komplex in Pyridin umgesetzt und dann direkt mit dem gewünschten Kation gegebenenfalls in Anwesenheit eines geeigneten Puffers zu dem gewünschten Salz umgesetzt werden.

Die erfindungsgemäßen pharmazeutischen Zusammensetzungen umfassen einen geeigneten Träger oder ein geeignetes Vehikel. Sie können darüber hinaus auch zusätzliche Wirkstoffe, wie andere Vitamine, Mineralstoffe und Spurenelemente sowie Arzneimittel jeglicher Art enthalten.

Geeignete Vehikel oder Träger für die transdermale Verabreichung sind alle dem Fachmann auf dem Gebiet der Pharmazie bekannten Vehikel für diesen Zweck. Dazu gehören flüssige oder feste Vehikel auf Fett- oder Ölbasis sowie Vehikel auf wässriger oder wässrig-alkoholischer Basis. Die Formulierungen können in Form von Salben und Ölen, Lotionen , Lösungen, die aufgesprüht werden können, Suspensionen und Emulsionen jeglicher Art und Pflastern vorliegen, die den Wirkstoff in einem Vehikel enthalten. Die Formulierungen können Penetrationsverstärker, wie Dimethylsulfoxid, Emulgatoren sowie jegliche weiteren in der Pharmazie üblichen Hilfsstoffe enthalten, wie Verdünnungsmittel, Geschmacksstoffe, Solubilisatoren, Gleitmittel, Suspendiermittel, Bindemittel, Konservierungsmittel,.

Geeignete Formulierungen für die transmukosale Verabreichung sind z.B. wässrige oder alkoholisch-wässrige Lösungen, die weitere Hilfsstoffe enthalten können, Suppositorien auf Fett-Basis, zur vaginalen Verabreichung geeignete Cremes, Gele, Pasten, Schäume oder Sprays und Pessare und Tampons sowie für die topische Verabreichung im Mund oder sublinguale Verabreichung geeignete feste Dosierungformen, einschließllich Lutschtabletten, die aktive Komponente in einer aromatisierten Grundlage enthalten, gewöhnlich Sucrose und Akaziengummi oder Tragant; Pastillen, die aktive Komponente in einer inerten Basis wie Gelatine oder Glycerin; und Kaugummis. Auch Formulierungen für die transmukosale Verabreichung können Penetrationsverstärker, wie Dimethylsulfoxid, Emulgatoren sowie jegliche weiteren in der Pharmazie üblichen Hilfsstoffe enthalten.

Zubereitungen in flüssiger Form für die parenterale Verabreichung durch intradermale, subkutane und intramuskuläre Injektion schließen Suspensionen, Lösungen oder Emulsionen in öligen oder wässrigen Vehikeln Suspensionen, Lösungen oder Emulsionen in öligen oder wässrigen Vehikeln ein. Die Zubereitungen können Formulierungsmittel wie Suspendier-, Stabilisier- und / oder Dispergiermittel enthalten. Alternativ kann der Wirkstoff in Pulverform vorliegen, der zur Konstitution mit einem geeigneten Vehikel, beispielsweise sterilem, pyrogenfreiem Wasser, vor dem Gebrauch gemischt wir.

Eine ausführliche Darstellung von Darreichungsformen, die für die Verwendung in der vorliegenden Erfindung geeignet sind, findet sich z.B. in Remington, The Science and Practice of Pharmacy, Ed. Allen, Loyd V. Jr, 22nd Edition, Pharmaceutical Press.

### Beispiele

### Beispiel 1 - Herstellung von Triethylammoniumcholecalciferolsulfat (Referenz)

1,21 g Pyridin-Schwefeltrioxid-Komplex (ca. 6,76 mMol mit SO₃ komplexiertes Pyridin; Sigma-Aldrich; ≥ 45 Gew.-% SO₃ laut Hersteller) und 1,21 g (3,14 mMol) Cholecalciferol (Sigma-Aldrich) wurden unter Stickstoffatmosphäre mit 6,5 ml Pyridin versetzt. Die entstandene Lösung wurde 1 h bei 58 °C heftig gerührt. Dann gab man 0,63 ml (0,456 g; 4,55 mMol) Triethylamin dazu und rührte weitere 20 min bei 58 °C.

Anschließend wurde die Reaktionsmischung mit einem Eisbad bei 0 °C gekühlt, 16,5 ml einer kalten Methanol-Trichlormethan(10:1 Vol. /Vol.) -Lösung wurden dazugegeben und man rührte 20 min.

Die Lösung wurde durch eine Glasfritte filtriert und am Rotationsverdampfer vom Lösungsmittel befreit. Der Rückstand wurde zur weiteren Reinigung noch zweimal mit Methanol-Trichlormethan(10:1 Vol./Vol.) -Lösung versetzt und am Rotationsverdampfer vom Lösungsmittel befreit, wodurch man 1,52 g (3,13 mMol; 99,7 %) Triethylammoniumcholecaliferolsulfat erhielt.

DSC (Kieselgel): R_{F} = 0,42 in Methanol-Trichlormethan (1:9 Vol./Vol.).

### Beispiel 2 - Herstellung von Ammoniumcholecalciferolsulfat (Referenz)

1,52 g (3,13 mMol) Triethylammoniumcholecaliferolsulfat wurden mit etwa 14 ml gesättigter Ammoniumacetat-Lösung versetzt, bis sich ein weißer Niederschlag von Ammoniumcholecalciferolsulfat bildete, der nach Stehen über Nacht mit einer Glasfritte abfiltriert und unter Kühlung mit Leitungswasser mit einer Temperatur von etwa 15 °C im Hochvakuum getrocknet wurde. Ausbeute: 1,74 g (3,10 mMol, 99 %).

Schmp.: 104-108 °C

### Beispiel 3 - Herstellung von Cholecalciferolsulfat-Natrium (Referenz)

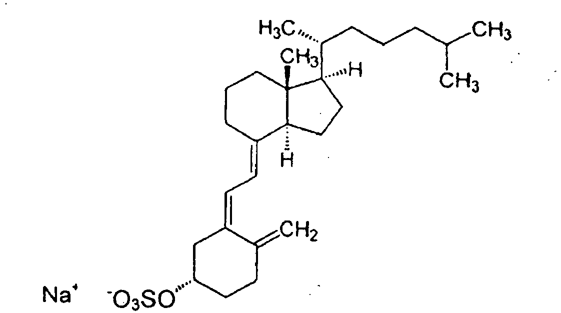

1,52 g (3,13 mMol) Triethylammoniumcholecaliferolsulfat wurden mit gesättigter Natriumchlorid-Lösung (ca. 14 ml) versetzt, bis sich ein weißer Niederschlag von Cholecalciferolsulfat-Natrium bildete, der nach Stehen über Nacht mit einer Glasfritte abfiltriert und unter Kühlung mit Leitungswasser mit einer Temperatur von etwa 15 °C im Hochvakuum getrocknet wurde. Ausbeute: 1,75 g (3,10 mMol, 99 %).

Das NMR-Spektrum war identisch mit dem in L. E. Reeve et al., The Journal of Biological Chemistry (1981) Bd. 256, Nr. 2, S. 824 veröffentlichten.

### Beispiel 4 - Herstellung von Cholecalciferolsulfat-Magnesium (Referenz)

1,52 g (3,13 mMol) Triethylammoniumcholecaliferolsulfat wurden mit gesättigter Magnesiumchlorid-Lösung (ca. 13 ml) versetzt, bis sich ein weißer Niederschlag von Cholecalciferolsulfat-Magnesium bildete, der nach Stehen über Nacht mit einer Glasfritte abfiltriert und unter Kühlung mit Leitungswasser mit einer Temperatur von etwa 15 °C im Hochvakuum getrocknet wurde. Ausbeute: 1,76 g (3,10 mMol; 99 %).

Schmp.: 107-110°C(Zers.).

### Beispiel 5 - Herstellung von Cholecalciferolsulfat-Calcium (Referenz)

1,52 g (3,13 mMol) Triethylammoniumcholecalciferolsulfat wurden mit gesättigter CaCl₂·2H₂O-Lösung (ca. 13 ml) versetzt, bis sich ein weißer Niederschlag von Cholecalciferolsulfat-Calcium bildete, der nach Stehen über Nacht mit einer Glasfritte abfiltriert und unter Kühlung mit Leitungswasser mit einer Temperatur von etwa 15 °C im Hochvakuum getrocknet wurde. Ausbeute: 1,81 g, (3,10 mMol, 99 %).

DSC (Kieselgel): R_{f} = 0,48 in Methanol-Trichlormethan (1:9 Vol./Vol.) Schmp.: 97-101°C (Zers.).

### Beispiel 6 - Herstellung von L-Lysincholecalciferolsulfat

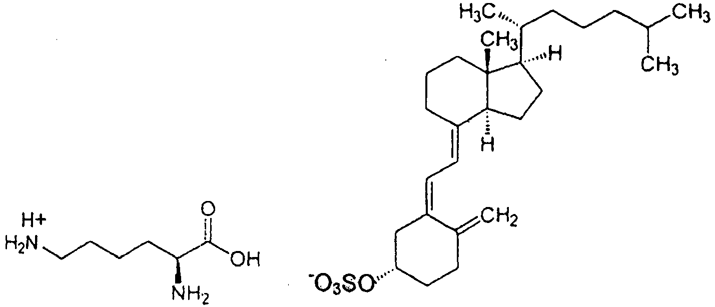

517,4 mg Pyridin-Schwefeltrioxid-Komplex (Sigma-Aldrich; ≥ 45 Gew.-% SO₃ laut Hersteller) (ca. 2,90 mMol mit SO₃ komplexiertes Pyridin) und 506,3 mg (1,32 mMol) Cholecaliferol (Sigma-Aldrich) wurden unter Stickstoffatmosphäre in 4,4 ml Pyridin gelöst und 1 h unter heftigem Rühren bei 55 °C erwärmt. Nach Zugabe von 10 ml eisgekühlter Methanol-Trichlormethan (1:9 Vol/Vol)-Mischung wurden die Lösungsmittel am Rotationsverdampfer entfernt. Dann wurden 0,29 g L-Lysin (1,98 mMol) in 0,5 ml Natriumphosphat-Pufferlösung, pH 7,3, dazugegeben und man rührte 5 min. Anschließend wurden 20 ml eisgekühlte Methanol-Trichlormethan (1:9 Vol/Vol)-Mischung unter Rühren dazugegeben, danach dampfte man die Mischung am Rotationsverdampfer ein. Zu dem Rückstand wurden 10 ml absolutes Ethanol gegeben und die Lösung wurde über Nacht im Kühlschrank aufbewahrt. Dann wurde das Ethanol von dem entstandenen weißen, cremigen Niederschlag abdekantiert und der Rückstand wurde unter Kühlung mit Leitungswasser mit einer Temperatur von etwa 15 °C getrocknet, wodurch man die Titelverbindung als weißes Pulver erhielt (815 mg, (1,30 mMol); 98,5 %).

DCS (Kieselgel): R_{f} = 0,33 in Methanol-Trichlomethan (1:9 Vol./Vol.).

Schmp.: 168°C (Zers.)

NMR-Spektrum: siehe Fig. 1

### Beispiel 7 - Herstellung von D,L-Lysincholecalciferolsulfat

Die Herstellung von D,L-Lysincholecalciferolsulfat erfolgte auf analoge Weise wie die Herstellung von L-Lysincholecalciferolsulfat unter Verwendung von DL-Lysin (Sigma.-Aldrich) anstelle von L-Lysin.

Das NMR-Spektrum in D4-Methanol bestätigte die Struktur.

### Beispiel 8 - Transdermale Formulierung von L-Lysincholecalciferolsulfat in einer Ölbasis

100 mg L-Lysincholecalciferolsulfat wurden mit 11,04 ml Ölsäure gemischt, dann wurden 0,83 ml Dimethylsulfoxid dazugegeben. Man rührte mit einem Magnetrührer 2 Tage bei Raumtemperatur (21-25 °C). Anschließend wurden 10 ml Glyceroltrioleat und 7 ml Glycerolmomonooleat (Pecerol®, Gattefosse) dazugegeben und man schüttelte heftig. Nachdem man gewartet hatte, bis die Mischung entschäumt war, erhielt man eine nahezu vollständig klare, stabile Lösung von L-Lysincholecalciferolsulfat in der Ölphase.

### Beispiel 9 - Auftragen der transdermalen Formulierung von Beispiel 8 auf die Haut

Ungefähr 2 ml der in Beispiel 8 hergestellten Formulierung wurden in dünner Schicht auf die Haut eines Probanden aufgetragen und 4 Stunden einziehen gelassen. Anschließend wurde die Haut mit einem sterilen Wattebausch, der mit 96%-igem Ethanol getränkt war, gut abgerieben. Der Wattebausch wurde mit weiterem mit 96%-igem Ethanol ausgekocht und ausgepresst, das Ethanol wurde am Rotationsverdampfer abgedampft und in den Kolben wurde wenig Chloroform-Methanol (9:1 Vol./Vol.)-Mischung gegeben. DSC dieser Mischung auf Kieselgel zeigte, dass praktisch kein L-Lysincholecalciferolsulfat (R_{f}: 0,33) auf der Haut verblieben war.

### Beispiel 10 - Herstellung wässriger Injektionslösungen

Zur Herstellung einer 2 ml-Injektionslösung, die Cholecalciferolsulfat-Salz in einer Menge enthält, die 10.000 IE Vitamin D3 entspricht, wurden
a) 1,5695 mg Cholecalciferolsulfat-Natrium zur Herstellung der Injektionslösung A
b) 1,6243 mg Cholecalciferolsulfat-Calcium zur Herstellung der Injektionslösung B und
c) 1,970 mg L-Lysincholecalciferolsulfat zur Herstellung der Injektionslösung C jeweils in 10 ml dest. Wasser gelöst. Dann wurden 89,9028 mg Natriumchlorid zu jeder Lösung gegeben, um sie isotonisch zu machen.

Der pH der Injektionslösungen B wurde mit 0,5n NaOH auf pH 7 gebracht. Anschließend wurden die Lösungen unter Argonatmosphäre durch eine 0,22 µm Membran filtersterilisiert und in 2 ml-Ampullen abgefüllt.

## Patentansprüche

1. Pharmazeutisch annehmbare Cholecalciferolsulfat-Salze, ausgewählt aus L-Lysincholecalciferolsulfat und D,L- Lysincholecalciferolsulfat.

2. Pharmazeutische Zusammensetzung, umfassend mindestens ein pharmazeutisch annehmbares Cholecalciferolsulfat-Salz nach Anspruch 1 und ein für die transdermale oder transmukosale Verabreichung oder die intradermale, subkutane oder intramuskuläre Injektion, einschließlich Depot- Injektionen mit verlängerter Wirkstofffreigabe, geeignetes Vehikel.

3. Pharmazeutische Zusammensetzung nach Anspruch 2, bei der das Vehikel ein für die transdermale Verabreichung geeignetes Vehikel auf Öl- oder Fettbasis ist.

4. Pharmazeutische Zusammensetzung nach Anspruch 2, bei der das Vehikel ein für die transdermale Verabreichung geeignetes Vehikel auf wässriger oder wässrig- alkoholischer Basis ist.

5. Pharmazeutische Zusammensetzung nach einem der Ansprüche 2 bis 4, die in Form eines Pflasters vorliegt.

6. Pharmazeutische Zusammensetzung nach Anspruch 2, bei der das Vehikel ein für die transmukosale Verabreichung geeignetes Vehikel auf wässriger oder wässrig- alkoholischer Basis ist.

7. Pharmazeutische Zusammensetzung nach Anspruch 2, bei der das Vehikel ein für die intradermale, subkutane oder intramuskuläre Injektion geeignetes Vehikel auf wässriger oder wässrig-alkoholischer Basis ist.

## Claims

1. Pharmaceutically acceptable cholecalciferol sulfate salt selected from L-lysine cholecalciferol sulfate and D, L-lysine cholecalciferol sulfate.

2. A pharmaceutical composition comprising at least one pharmaceutically acceptable cholecalciferol sulfate salt of claim 1 and a vehicle suitable for transdermal or transmucosal administration or intradermal, subcutaneous or intramuscular injection, including sustained release injections.

3. The pharmaceutical composition according to claim 2, wherein the vehicle is an oil or fat based vehicle suitable for transdermal administration.

4. The pharmaceutical composition according to claim 2, wherein the vehicle is an aqueous or aqueous-alcoholic vehicle suitable for transdermal administration.

5. The pharmaceutical composition according to any of claims 2 to 4 which is in the form of a plaster.

6. The pharmaceutical composition according to claim 2, wherein the vehicle is an aqueous or aqueous-alcoholic vehicle suitable for transmucosal administration.

7. The pharmaceutical composition according to claim 2, wherein the vehicle is an aqueous or aqueous-alcoholic vehicle suitable for intradermal, subcutaneous or intramuscular injection.

## Revendications

1. Sels de sulfate de cholécalciférol pharmaceutiquement acceptables choisis parmi le sulfate de cholécalciférol de L-lysine et le sulfate de cholécalciférol de D,L-lysine.

2. Composition pharmaceutique comprenant au moins un sel de sulfate de cholécalciférol pharmaceutiquement acceptable selon la revendication 1 et un véhicule approprié pour une administration transdermique ou transmuqueuse ou une injection intradermique, sous-cutanée ou intramusculaire, y compris des injections à libération prolongée.

3. Composition pharmaceutique selon la revendication 2, dans laquelle le véhicule est un véhicule à base d'huile ou de graisse approprié pour l'administration transdermique.

4. Composition pharmaceutique selon la revendication 2, dans laquelle le véhicule est un véhicule aqueux ou hydroalcoolique convenant à l'administration transdermique.

5. Composition pharmaceutique selon l'une quelconque des revendications 2 à 4, qui se présente sous la forme d'un emplâtre.

6. Composition pharmaceutique selon la revendication 2, dans laquelle le véhicule est un véhicule aqueux ou hydroalcoolique adapté à une administration transmuqueuse.

7. Composition pharmaceutique selon la revendication 7, dans laquelle le véhicule est un véhicule aqueux ou hydroalcoolique adapté à l'injection intradermique, sous-cutanée ou intramusculaire.
